(19) Europäisches Patentamt
European Patent Office
Office européen des brevets

(11) Publication number: 0 195 053 B1

(12) EUROPEAN PATENT SPECIFICATION

(45) Date of publication of patent specification: 12.02.92

(51) Int. Cl.⁵: C07C 45/72, C07C 45/66, C07C 49/00, C07C 321/12

(21) Application number: 85904773.0

(22) Date of filing: 26.09.85

(86) International application number: PCT/US85/01853

(87) International publication number: WO 86/02065 (10.04.86 86/08)

The file contains technical information submitted after the application was filed and not included in this specification

# (54) PROCESS FOR PREPARING 1,3-CYCLOHEXANEDIONE DERIVATIVES AND INTERMEDIATES THEREFOR.

(30) Priority: 27.09.84 US 655776

(43) Date of publication of application: 24.09.86 Bulletin 86/39

(45) Publication of the grant of the patent: 12.02.92 Bulletin 92/07

(84) Designated Contracting States: CH DE FR GB LI NL

(56) References cited:
DE-C- 840 090
FR-A- 2 481 701
GB-A- 783 458
GB-A- 2 125 042
US-A- 4 355 184

Chemical Abstracts, volume 94, 1981, Columbus, Ohio (US), see page 638, abstract no. 120871x

(73) Proprietor: CHEVRON RESEARCH AND TECHNOLOGY COMPANY
P.O. Box 7643
San Francisco, CA 94120(US)

(72) Inventor: CLEVELAND, James, D.
724 Cerrito Street
Albany, CA 94706(US)

(74) Representative: Kosmin, Gerald Emmanuel et al
HASELTINE, LAKE & CO. Hazlitt House 28 Southampton Buildings Chancery Lane
London, WC2A 1AT(GB)

EP 0 195 053 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing alpha,beta-unsaturated ketones and substituted 1,3-cyclohexanediones. The aforementioned compounds are useful as intermediates for medicaments and pesticides, especially herbicides.

For example, the alpha,beta-unsaturated ketones are useful for preparing the 2-oximino-5-substituted-cyclohexane-1,3-dione class of herbicides via the following known reaction route

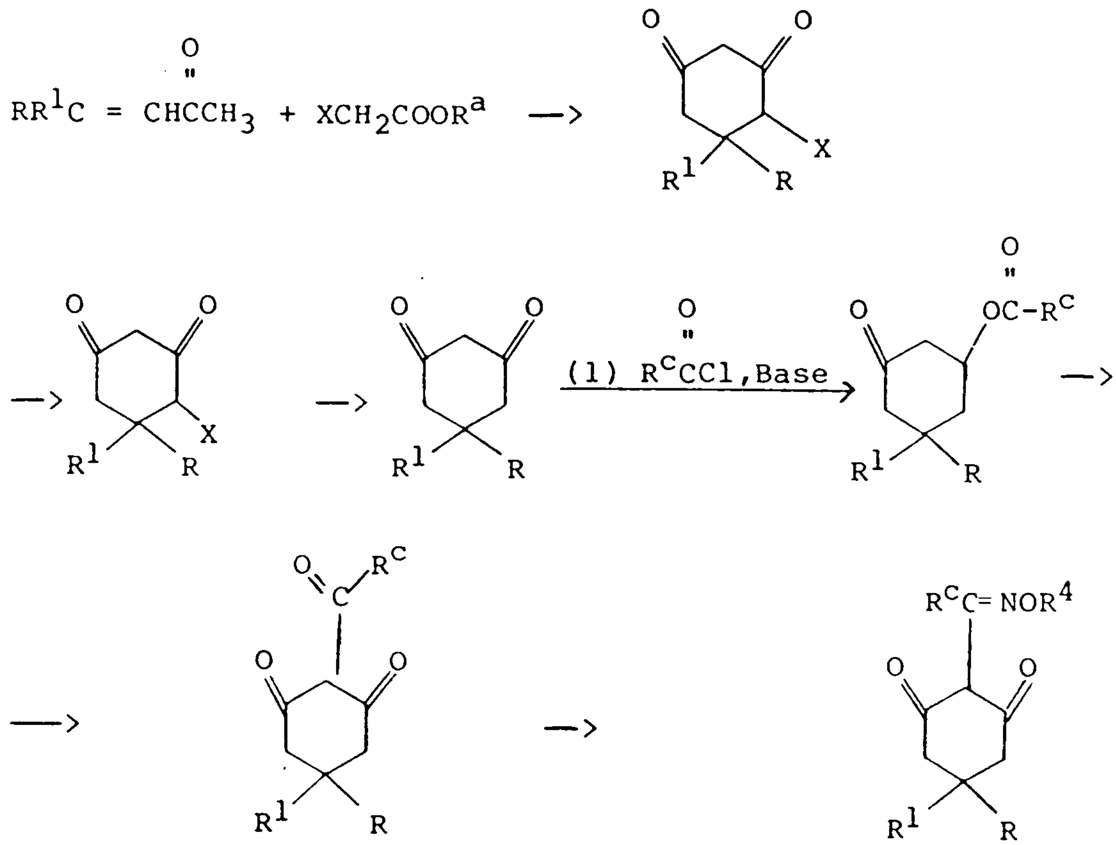


wherein R is a variety of organic residues, $R^1$ is hydrogen or organic residue, $R^a$ is lower alkyl, $R^c$ is lower alkyl, $R^4$ is alkyl, alkenyl, haloalkenyl, etc.; X is cyano, nitro or $-COOR^b$, wherein $R^b$ is lower alkyl.

Such herbicides are, for example, described in U.S. Patents Nos. 3,950,420; 3,989,737; 4,011,256; 4,249,937; 4,334,913; 4,440,566; 4,422,864; German Patent Applications DE 3219315; DE 3227332; DE 3227389; European Patent Application Publication 046860 and published Japanese Patent Application J51013755 (Derwent Abstract No. 21456X/12).

U.S. Patent No. 4,355,184 describes a process for preparing alpha,beta-unsaturated ketones via the reaction of an aldehyde with an alkali metal salt of acetoacetic acid in the presence of an aliphatic secondary amine in a mixed heterogeneous solvent system.

Based on the art description given in U.S. Patent No. 4,355,184, the synthesis of alpha,beta-unsaturated ketones via the aldol condensation of aldehydes and acetone is described in Ber. 40, 4764 (1907). Indian J. Chem. Vol. 16B, 970 (1978) describes the condensation of aldehydes with ketones in the presence of a piperidineacetic acid catalyst system. Ber. Vol. 103 2077 (1970) describes the synthesis of alpha,beta-unsaturated ketones via the reaction of an aldehyde with Wittig reagent. Acta. Chem. Scand. v. 17, 2216 (1963) describes the production of such ketones via the decomposition of alpha,beta-unsaturated-beta'-ketoic esters with heat in the presence of p-toluene sulfonic acid. U.S, Patent No. 2,108,427 describes the synthesis of alpha,beta-unsaturated ketones via the reaction of an aldehyde with a diketene.

German patent 840,090 describes a procedure for preparing unsaturated ketones by dehydrating beta-hydroxy ketones to the corresponding unsaturated ketones, by heating in the presence of oxalic acid in aqueous medium.

Japanese Patent No. 55-141429 (as reported in Chem.Abs.94(1981) 638,120871X), teaches the preparation of beta hydroxy ketones, by the reaction of an appropriately substituted aldehyde with sodium aceto-acetate in methanol in the presence of a tertiary amine. The resulting product, after acidification, is 2-keto-4-hydroxy-6-ethylthioheptane.

British Patent No. 783458 discloses that condensation between aldol-type reactants can be effected continuously using as a catalyst a volatile tertiary amine and that monohydroxy compounds produced thereby can be converted into ethylenically unsaturated compounds by dehydration in the presence of a dehydration catalyst.

It has now surprisingly been found that certain α,β -unsaturated aldehydes and ketones useful in the production of substituted 1,3-cyclohexanediones employed as intermediates for preparing the 2-oximino-5-substituted-cyclohexane-1,3-dione class of herbicides can be prepared in improved yield by a two-step process of the type generally disclosed in the aforementioned British patent, rather than by a one-step process such as that disclosed in the aforementioned U.S. patent, so that higher yields of the 1,3-cyclohexanedione intermediates can consequentially be obtained.

Thus, in accordance with the invention, there is provided a process for preparing a 4,5-disubstituted-1,3-cyclohexanedione represented by the general formula:

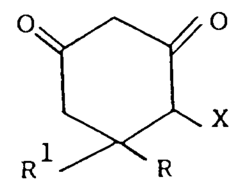

wherein X is cyano, nitro or $COOR^b$ where $R^b$ is lower alkyl; R is lower alkyl, cycloalkyl having 3 to 7 carbon atoms, lower alkoxy, haloalkyl having 1 or 2 carbon atoms and 1 to 3 of the same or different halo atoms, aryl having 6 to 10 carbon atoms; substituted aryl having 6 to 10 carbon atoms in the aryl moiety and having 1 to 3 substituents independently selected from lower alkyl, lower alkenyl, lower alkoxy, lower alkylthio, halo, haloalkyl having 1 or 2 carbon atoms and 1 to 3 of the same or different halo atoms, and nitro; arylalkyl having 1 to 4 carbon atoms in the alkyl moiety and 6 to 10 carbon atoms in the aryl moiety; substituted arylakyl wherein the alkyl moiety has 1 to 4 carbon atoms and the substituted aryl moiety is as defined hereinabove; monocyclic heterocyclic or heterocyclicalkyl having 1 to 4 carbon atoms in the alkyl moiety and wherein the heterocyclic moiety has 5- or 6-ring atoms, 1 or 2 of which are independently oxygen, sulfur or nitrogen atoms, and the remainder are carbon atoms; or R is the radical $R^5S(0)_nR^4$ - where $R^4$ is lower alkylene, $R^5$ is lower alkyl, lower alkenyl, cycloalkyl as defined hereinabove, aryl, substituted aryl as defined hereinabove, or arylalkyl or substituted arylalkyl as defined hereinabove and n is 0, 1 or 2; and $R^1$ is hydrogen or a substituent as defined for R, or R and $R^1$ together with the carbon atom to which they are joined form a cycloalkylidene group having 5 to 7 carbon atoms, which comprises reacting in a known manner an α,β -unsaturated ketone of the general formula:

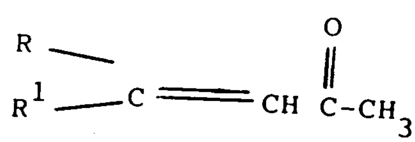

wherein R and $R^1$ have the meanings hereinbefore defined, with a substituted acetate ester of the general formula:

$XCH_2\ COOR^a$

wherein $R^a$ is lower alkyl and X has the meaning hereinbefore defined, in the presence of an alkali metal lower alkoxide to obtain the desired compound, if necessary by means of a decarboxylation step, characterised in that the α,β -unsaturated ketone starting compound is produced by a process which comprises the separate steps of:

(a) contacting an aldehyde or ketone of the general formula:

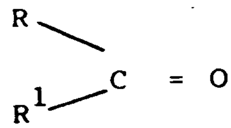

wherein R and $R^1$ have the meanings hereinbefore defined, with an acetoacetate salt in the presence of a catalytic amount of a tertiary amine to form a compound of the general formula:

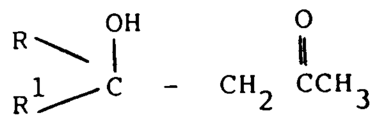

wherein R and $R^1$ have the meanings hereinbefore defined, and thereafter

(b) contacting said compound with a dehydration catalyst at a temperature in the range from 60 to 200°C to produce the required $\alpha,\beta$-unsaturated starting compound.

In the process of the invention, an appropriate aldehyde (RCHO) or ketone [RC(0)$R^1$] is reacted with an acetoacetate salt in the presence of a tertiary amine to yield the corresponding 4-substituted-4-hydroxyalkan-2-one. This product is in turn dehydrated to yield the corresponding alpha,beta-unsaturated ketone product mixture.

The process can be schematically illustrated by the following overall reaction equations:

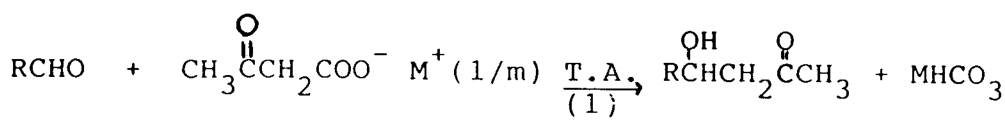


(A) (B) (C)

$$RCH(OH)CH_2COCH_3 \xrightarrow[(2)]{\text{dehydration}} RCH{=}CHC(O)CH_3 + H_2O$$

(C) (D)

wherein R has the meaning hereinbefore defined; M is a cation and m is its valence; and T.A. is a tertiary amine.

The first step of this process can be effected by contacting aldehyde (A) with acetoacetate salt (B), preferably in an aqueous solution, in the presence of a catalytic amount of a tertiary amine, and optionally in an inert organic solvent, under reactive conditions.

Typically, this process is conducted at temperatures in the range from 20 to 65°C, preferably 50 to 55°C, for from 2 to 8 hours, preferably 2 to 4 hours, using from 1 to 1.5 moles equivalents, preferably 1 to 1.3 moles equivalents of compound B and 0.01 to 0.1 moles, preferably 0.03 to 0.05 moles, of tertiary amine per mole of compound A. Preferably, the process is conducted at pH's in the range from 8 to 10, more preferably 8.2-8.6.

Suitable tertiary amines which can be used include, for example, trialkylamines, e.g., triethylamine, methyl diethylamine, nitrogen heterocycles, e.g., N-methylpiperidine, pyridine, etc., and the like.

Where an inert organic solvent is used, suitable solvents which can be used include, for example, liquid alkanols (e.g. methanol, ethanol, etc.) aromatic hydrocarbons, e.g., benzene, toluene, chlorinated hydrocarbons, e.g., methylene chloride, chloroform, alkanes, e.g., hexane, heptane, and the like and compatible mixtures thereof. Also, as before noted an aqueous solution of salt B is preferably used. Typically, the aqueous solution has a salt (B) concentration in the range from 5 to 30, preferably 10 to 15 weight percent.

Also, if it is desired to produce an $RR^1C{=}CHC(0)CH_3$ product (D'), wherein $R^1$ is an organic substituent as hereinbefore defined, the corresponding RC(0)$R^1$ ketone (A') can be used in the above process in place of aldehyde A. For example, by using acetone, $CH_3(CH_3)C{=}CHC(0)CH_3$ is produced. However, best results are generally obtained using aldehydes.

The aldehyde starting materials (A) and ketones (A') are generally known compounds and can in any case be prepared by adapting known procedures using the appropriately substituted starting materials.

As before noted, the R group in compounds A, C, and D (and also the $R^1$ group, if a ketone is used) include lower alkyl; cycloalkyl having 3 to 7 carbon atoms; aryl generally having 6 to 10 carbon atoms; substituted aryl generally having 6 through 10 carbon atoms in the aryl moiety and 1 to 3 substituents independently selected from lower alkyl, lower alkenyl, lower alkoxy, lower alkylthio, halo, haloalkyl having 1 or 2 carbon atoms and having 1 to 3 of the same or different halo atoms, and nitro; arylalkyl generally having 1 to 4 carbon atoms in the alkyl moiety and 6 to 10 carbon atoms; substituted arylalkyl wherein generally the alkyl moiety has 1 to 4 carbon atoms and the substituted aryl moiety is as defined hereinabove; heterocyclic radicals and heterocyclic-alkyl radicals generally having 1 to 4 carbon atoms in the alkyl moiety and wherein the heterocyclic moiety has 5- or 6-ring atoms, 1 or 2 of which are independently oxygen, sulfur or nitrogen atoms, preferably oxygen or sulfur, and the remainder are carbon atoms; or R is the radical $R^5S(0)_nR^4$- wherein $R^4$ is lower alkylene and $R^5$ is lower alkyl, lower alkenyl, cycloalkyl as defined hereinabove, aryl or substituted aryl generally as defined hereinabove, or arylalkyl or substituted arylalkyl and n is 0, 1 or 2. Preferably $R^5$ is lower alkyl, so that R may advantageously be the radical

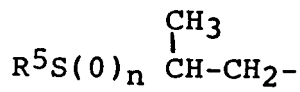

where $R^5$ is lower alkyl, preferably the radical 2-ethylthiopropyl. Where a ketone is used $R^1$ can be independently selected from the group indicated for R or $R^1$ and R together with the carbon atom to which they are joined can form a cycloalkylidene having 5- or 6-ring carbon atoms.

As above noted with respect to salt B, M is a cation. Preferred M cations are those cations which yield water soluble salts (B) and include, for example, the cations of alkali metals, e.g., sodium, and potassium, etc.

The second step, dehydration, can be effected by contacting compound C with a suitable dehydration catalyst at elevated temperatures, optionally in an inert organic solvent or water.

This process is conducted at temperatures in the range from 60 to 200°C, preferably 80 to 150°C, generally for from 1 to 3 hours, preferably 1 to 1.5 hours, generally using from 0.03 to 0.15, preferably 0.04 to 0.08 mole equivalents of dehydration catalyst per mole of compound C. The optimum amount of dehydration catalyst can vary with the particular catalyst used, but can be determined by routine experimentation.

Typical dehydration catalysts which can be used include, for example, dicarboxylic acids, typically having 3 to 6 carbon atoms, such as for example, oxalic acid, malonic acid, or substituted acetic acids whose pKa values approximate oxalic acid, and the like. We have found that best results are generally obtained using oxalic acid.

If desired, this process can be conducted in an inert organic solvent or water. However, very good results are obtained even without a solvent. Where an inert organic solvent is used, suitable solvents which can be used include, for example, aromatic hydrocarbons, for example, toluene, xylene, and the like and compatible mixtures thereof. Best results are generally obtained using no solvent or using toluene or xylene as the solvent. Where the reaction is conducted neat (i.e., without a solvent), the reaction is preferably conducted at temperatures in the range of from 100 to 115°C. The process can also be conveniently conducted in toluene-water azeotrope or xylene-water azeotrope in which case the process is preferably conducted at temperatures in the range from 85 to 115°C and 90 to 145°C, respectively. The azeotrope is formed by the water which is generated as a byproduct of the dehydration reaction.

The present process provides high yields, typically total yields of 90% or better, based on aldehyde A or ketone A', of alpha,beta-unsaturated ketone. Typically, the product is a mix of *trans* and *cis* isomer, with the major isomer typically being the *trans* isomer. The crude reaction product (*i.e.,* the reaction product after removal of solvent) also contains *a* small amount, generally less than 5 wt. % of ketone adduct, i.e.,

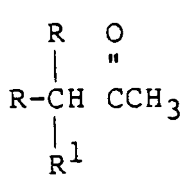 or 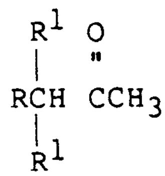

wherein R and $R^1$ are as defined hereinabove. For example, where 6-ethylthiobutrylaldehyde is used, the adduct is 2,6-di(ethylthio)heptan-2-one. The crude reaction product also contains a small amount, typically less than 4 wt. %, of the substituted 4-hydroxybutan-2-one intermediate C. However, neither product detracts from the usefulness of the crude product for the preparation of 1,3-cyclohexanedione derivatives. Because both products in low concentration are converted to the 1,3-cyclohexanedione along with both the cis and trans alpha,beta-unsaturated ketone isomers. It is noted that the 4-hydroxyalkan-2-one intermediate C cannot by itself be used to prepare the cyclohexanedione because the reaction generates byproduct water which would ultimately poison the reaction. However, because of the small amount of intermediate C in the present crude product, the correspondingly small amount of byproduct water generated in the cyclization reaction is easily tolerated by the reaction.

Thus, the present process provides an especially useful intermediate for the production of 1,3-cyclohexanedione derivatives.

The 1,3-cyclohexanedione cyclization can be represented by the following overall reaction equation:

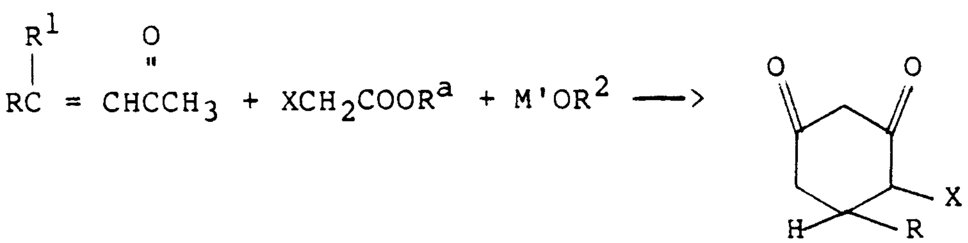

(D) (E) (F) (G)

wherein R and $R^1$ is as defined hereinabove; M' is a cation preferably alkali metal, e.g. sodium; $R^a$ and $R^2$ are independently lower alkyl, X is cyano, nitro or $-COOR^b$, wherein $R^b$ is lower alkyl.

This process can be effected by contacting product D with compound E and an alkanolate salt (F), preferably in an inert organic solvent.

Typically, this process is conducted at temperatures in the range of about from 15 to 70 °C, preferably 20 to 40 °C, for about from 3 to 10 hours, preferably 3 to 7 hours, using about from 1.0 to 1.5 moles, preferably 1.0 to 1.2 moles of compound E and 1.0 to 1.5 moles, preferably 1.0 to 1.2 moles of compound F per mole of compound D mixture.

Suitable inert organic solvents which can be used, include, for example, alcohols, e.g., methanol, ethanol, and aromatic hydrocarbons, e.g., benzene and toluene, and the like and compatible mixtures thereof. Suitable substituted acetate ester which can be used, include, for example, dimethylmalonate; methyl cyanoacetate; methyl nitroacetate, and the like. Where it is desired to ultimately produce a product which is unsubstituted at the 4-position, then typically, dimethyl malonate or diethyl malonate is used as they are readily commercially available, relatively inexpensive, and give good results.

Where it is desired to prepare a product which is unsubstituted at the 4-position, it is preferred to prepare the 4-alkoxycarbonyl (i.e., X is $-COOR^b$) compound of formula G. In this case, the 4-substituent can be readily removed by first base hydrolyzing off the alkoxy group, yielding the carboxy group or base carboxy salt, followed by decarboxylation to remove the 4-carboxy group.

The base hydrolysis can be conveniently effected by contacting compound G' with a base (e.g., sodium hydroxide, potassium hydroxide) typically at a pH of from 12 to 14 and temperatures of 20 to 40 °C for from 1 to 3 hours. The resulting product is decarboxylated by contact with an acid (e.g., hydrochloric acid, sulfuric acid, etc.). The reaction is typically conducted at a pH of from 4 to 4.5 for from 1 to 4 hours at temperatures in the range of 20 to 100 °C.

An especially useful and valuable application of this invention is in the production of 2-keto-6-ethylthioheptene-3 which in turn gives improved yields of 5-substituted- and 4-carboalkoxy-5-substituted cyclohexane-1,3-diones. In some cases, the compounds are also obtained in higher purity. In this aspect, 3-ethylthiobutyraldehyde is reacted with a salt of acetoacetic acid in an inert organic solvent with the pH adjusted within the range of 6 to 9 by the addition of an acid, in the presence of a catalytic amount of a tertiary amine. The resulting crude product is then contacted with a catalytic amount of oxalic acid to produce the desired 2-keto-6-ethylthiohept-3-ene in high purity and high yield.

In a preferred embodiment of this aspect of the invention, the first step of the process is conducted using an aqueous solution of sodium acetoacetate acidified to pH 8.2-8.6 by the addition of a suitable acid, such as, for example, hydrochloric acid or other mineral acid. An inert organic solvent, for example, methanol or ethanol is added, followed by the addition of a catalytic amount of the tertiary amine. 3-Ethylthiobutyraldehyde is then added slowly while the mixture is stirred at a temperature in the range of 20 to 25°C. After all the aldehyde has been added and stirred at 20-25°C for 1/2 to 1-1/2 hours, heating and then heated at 45-55°C for 2 to 4 hours or until analysis shows the reaction to be 90-95% complete. Then a water immiscible solvent, for example, toluene is added followed by concentrated hydrochloric acid to pH of about 4. The aqueous and organic layers are separated. The crude product toluene layer is then mixed with about 5 mole % of oxalic acid based on starting aldehyde. The resulting mixture is heated at temperatures ranging from about 85 to 119°C, with azeotropic removal of water. The solvent may be optionally codistilled with water so the dehydration is done neat typically with an improved reaction time of less than one hour. This heating is continued until analysis indicates less than 2% of the feed 6-ethylthio-4-hydroxyheptan-2-one remains. Work-up can be effected by dissolution in solvent, e.g., toluene, washing with aqueous sodium bicarbonate followed by azeotropic drying. Finally, the toluene is removed by distillation to leave the crude product.

The dehydration step of the instant process can be carried out neat with about 5% of the oxalic acid catalyst, with no solvent, and at a reaction time of less than one hour. The crude reaction product is a mixture of *trans* and *cis* 2-keto-6-ethylthiohept-3-ene. I have found that the crude product after removal of catalyst and drying can be reacted directly with dialkyl malonate, e.g., dimethyl malonate, in the presence of a base, such as sodium methoxide, to give very good yields of 4-carbalkoxy-5-(2-ethylthiopropyl)-cyclohexane-1,3-dione.

## General Process Conditions

In the above-described processes, it is generally preferable to separate the respective products before proceeding with the next step in the reaction sequence, except where described as an *in situ* step or unless otherwise expressly stated. These products can be recovered from their respective reaction product mixtures by any suitable separation and purification procedure, such as, for example, extraction, distillation or chromatography. Suitable separation and purification procedures are, for example, illustrated in the Examples set forth hereinbelow.

Generally, the reactions described above are conducted as liquid phase reaction and hence pressure is generally not significant except as it affects temperature (boiling point) where reactions are conducted at reflux. Therefore, these reactions are generally conducted at pressures of from 300 to 3,000 mm of mercury and conveniently are conducted at atmospheric or ambient pressure.

It should also be appreciated that where typical or preferred process conditions (e.g., reaction temperatures, times, mole ratios of reactants, solvents, etc.) have been given, that other process conditions could also be used. Optimum reaction conditions (e.g., temperature, reaction time, mol ratios, solvents, etc.) may vary with the particular reagents or organic solvents used but can be determined by routine optimization procedures.

## Definitions

As used herein the following terms have the following meanings unless expressly stated to the contrary:

The term "lower alkyl" refers to both straight-and branched-chain alkyl groups having a total of from 1 through 6 carbon atoms, preferably 1 through 4 carbon atoms and includes primary, secondary and tertiary alkyl groups. Typical lower alkyls include, for example, methyl, ethyl, n-propyl, isopropyl, n-butyl, t-butyl.

The term "lower alkylene" refers to both straight chained and branched chained alkylene groups having 1 through 6 carbon atoms, preferably 1 through 4 carbon atoms and includes

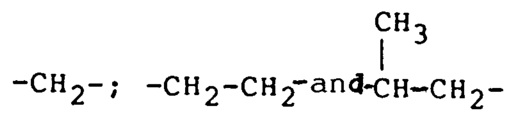

The term "lower alkenyl" refers to alkenyl groups having 2 through 6, preferably 2 through 4, carbon atoms and includes, for example, vinyl, 1-propenyl, 2-propenyl, 1-methylvinyl, 1-butenyl, 2-methylprop-1-enyl and the like.

The term "lower alkoxy" refers to the group -OR' wherein R' is lower alkyl.

The term "lower alkylthio" refers to the group -SR' wherein R' is lower alkyl.

The term "lower alkoxyalkyl" refers to the group R'OR''- wherein R' and R'' are independently straight chain or branched chain alkyl groups having 1 through 3 carbon atoms.

The term "lower alkylthioalkyl" refers to the group R'SR''- wherein R' and R'' are independently straight chain or branched chain alkyl groups having 1 through 3 carbon atoms.

The term "lower alkoxycarbonylalkyl" refers to the group

$$R'O\overset{\overset{O}{\|}}{C}R''-$$

wherein R' is lower alkyl, preferably having 1 through 4 carbon atoms, and R'' is alkylene having 1 through 4 carbon atoms and can be straight or branched chained. Typical alkoxycarbonylalkyl groups include for example, $-CH_2C(O)OCH_3$; $-CH(CH_3)C(O)OC_2H_5$, and the like.

The term "halo" refers to the group of fluoro, chloro, bromo and iodo.

The term "lower haloalkyl" refers to haloalkyl compounds having 1 through 4 carbon atoms and 1 through 3 halo atoms independently selected from the group of fluoro, chloro, bromo and iodo. Preferably the lower haloalkyl group has 1 or 2 carbon atoms.

The term "lower haloalkoxy" refers to "lower alkoxy" groups having 1 through 3 halo atoms independently selected from the group of fluoro, chloro, bromo or iodo.

The term "aryl" refers to aryl groups having 6 through 10 carbon atoms and includes, for example, phenyl, naphthyl, indenyl. Typically the aryl group will be phenyl or naphthyl as compounds having such groups are more readily available commercially than other aryl compounds.

The term "substituted aryl" refers to aryl groups having 1 through 3 substituents independently selected from the group of lower alkyl, lower alkoxy, halo, nitro, or haloalkyl having 1 through 4 carbon atoms and 1 through 3 halo atoms. Typical substituted aryl groups include, for example, 2-fluorophenyl, 2-chlorophenyl, 2,6-dimethylphenyl, 4-fluorophenyl, 2-methylphenyl, 2-chloro,3-chloromethylphenyl, 2-nitro,5-methylphenyl, 2,6-dichlorophenyl, 3-trifluoromethylphenyl, 2-methoxyphenyl, 2-bromonaphth-1-yl, 3-methoxyinden-1-yl, and the like.

The term "arylalkylene" refers to the group $ArR^3$- wherein Ar is aryl and $R^3$ is alkylene having 1 through 3 carbon atoms and includes both straight-chained and branched-chained alkylenes, for example, methylene, ethyl, 1-methylethyl, and propyl.

The term "(substituted aryl)alkylene" or "ring-substituted arylalkylene" refers to the group $Ar'R^3$- wherein Ar' is substituted aryl and $R^3$ is alkylene as defined with respect to arylalkylene.

The term "room temperature" or "ambient temperature" refers to 20-25° C.

A further understanding of the invention can be had in the following non-limiting Preparation(s) and Example(s). Wherein, unless expressly stated to the contrary, all temperatures and temperature ranges refer to the Centigrade system and the term "ambient" or "room temperature" refers to 20-25° C. The term "percent" or "%" refers to weight percent and the term "mole" or "moles" refers to gram moles. The term "equivalent" refers to a quantity of reagent equal in moles, to the moles of the preceding or succeeding reactant recited in that example in terms of finite moles or finite weight or volume. Where given, proton-magnetic resonance spectrum (p.m.r. or n.m.r.) were determined at 90 mHz, signals are assigned as singlets (s), broad singlets (bis), doublets (d), double doublets (dd), triplets (t), double triplets (dt), quartets (q), and multiplets (m); and cps refers to cycles per second. Also where necessary examples are repeated to provide additional starting material for subsequent examples.

Preparation 1

3-Ethylthiobutyraldehyde

In this example, 1 mole of ethyl mercaptan was added dropwise to a mixture of 1 mole of crotonaldehyde and 0.005 moles of triethylamine at room temperature. After about 2 hours the mixture was slightly concentrated by evaporation under vacuum to remove any excess mercaptan and the title compound is afforded as a liquid.

Example 1

6-Ethylthio-4-hydroxyheptan-2-one

A sodium hydroxide solution containing 50.4 g of sodium hydroxide in 117.6 g of water was added

dropwise to a mixture containing 139.32 g methyl acetoacetate and 168 g of water at room temperature. After addition of the sodium hydroxide solution, the mixture was heated at 35°C for 6 hours affording a solution of sodium acetoacetate. The pH of the sodium acetoacette solution was adjusted to pH 8.3 by the addition of aqueous 9 wt. % hydrochloric acid. 200 ml of methanol was then added followed by the addition of 3 g of triethylamine. The entire 3-ethylthiobutyraldehyde reaction product from Example 1 was then added dropwise and stirred for one hour at room temperature. The reaction mixture was heated at 50-55°C over a period of time. During this period, the progress of the reaction was monitored by gas liquid chromatography and recorded in graph area percents. The results of this analysis is recorded in Table 1 hereinbelow. For convenience in recording, the compounds have been assigned the code numbers indicated in Table A.

TABLE A

| Code No. | Compound |
|---|---|
| 1 | 3-ethylthiobutyraldehyde |
| 2 | 6-ethylthio-4-hydroxyheptan-2-one |
| 3 | 4,6-di(ethylthio)-heptan-2-one |

TABLE 1

Gas Liquid Chromatographic Analysis of Reaction Mixture

| Time | °C*1 | COMPOUND CODE NO. 1 | 2 | 3 |
|---|---|---|---|---|
| 0 | 23 | 100 | - | - |
| 1 hr. | 54 | 9.3 | 79.6 | 1.6 |
| 2 hr. | 54 | 4.1 | 88.8 | 1.1 |
| 2.5 hr. | 53 | 4.3 | 89.1 | 1.2 |
| 3 hr. | 52 | 3.9 | 89.9 | 1.3 |
| 3.5 hr. | 52 | 3.2 | 90.5 | 1.5 |

*1 Temperature of reaction mixture at time sample was taken for gas chromatography analysis.

Example 3

6-Ethylthiohept-3-en-2-one

In this example, 4.5 g of oxalic acid was added to a solution containing 152.2 g (0.8 mole) of 6-ethylthio-4-hydroxyheptan-2-one, prepared according to the procedure of Example 1 in 100 ml of toluene and then refluxed. The progress of the reaction mixture was monitored by gas chromatography.

After reaction was complete, toluene was added and the solution washed with 200 ml of aqueous 2.5 wt. % sodium bicarbonate solution and separated. The toluene solution was azeotropically dried and found to contain 0.784 moles of alpha,beta-unsaturated ketone corresponding to a 98% yield based on the 6-ethylthioheptan-2-one starting material. On a solvent-free base the crude product was analyzed by gas-liquid chromatography using external standards as containing 87.3 wt. % 6-ethylthiohept-3-en-2-one (trans and cis isomers); 4.28 wt. % 4,6-di(ethylthio)heptan-2-one; 2.02 wt. % 4-hydroxy-6-ethylthioheptan-2-one.

Similarly, by applying the procedures of Examples 1 and 2 using the corresponding aldehyde or ketone starting material in Example 1 the following compounds can be prepared:
2-keto-6-ethylsulfonylhept-3-ene;
2-keto-6-ethylsulfinylhept-3-ene;
2-keto-3-hexane;
2-keto-4-cyclohexylbut-3-ene;
2-keto-5-cyclopentylpent-3-ene;
2-keto-4-phenylbut-3-ene;
2-keto-4-(2-methylphenyl)but-3-ene;
2-keto-4-(3,5-dichlorophenyl)but-3-ene;
2-keto-4-(3-trifluoromethylphenyl)but-3-ene;
2-keto-4-(4-nitrophenyl)but-3-ene;
2-keto-6-ethoxyhept-3-ene;
2-keto-5-(2-methoxyphenyl)pent-3-ene;
2-keto-5-(3-methylthiophenyl)pent-3-ene;
2-keto-5-(4-allylphenyl)pent-3-ene;
2-keto-6-(3-trifluoromethylphenyl)hex-3-ene;
2-keto-5-tetrahydropyran-4-yl)pent-3-ene;
2-keto-5-(1,3-dioxan-2-yl)pent-3-ene;
2-keto-5-(1,3-dithiolan-2-yl)pent-3-ene;
2-keto-5-(thien-2-yl)pent-3-ene;
2-keto-7-(1,3-dioxan-2-yl)hept-3-ene;
2-keto-4-methylpent-3-ene; and
2-keto-3-cyclohexylidene-propane.

Example 3

5-(2-Ethylthiopropyl)-1,3-cyclohexanedione

The title compound can be prepared by the following procedure.

In this example 124.17 g of dimethyl malonate (0.94 moles) are added to 228.5 g of 25 wt. % sodium methoxide (1.06 moles) in methanol. Crude 6-ethylthiohept-3-en-2-one, prepared according to the procedure of Example 2, containing 0.784 moles of active ingredients, is added portionwise to the sodium methoxide solution at 20 to 30 °C, over a period of one-half hour. The mixture was stirred at room temperature for 7 hours. During this period conversion to 4-methoxycarbonyl-5-(2-ethylthiopropyl)-1,3-cyclohexanedione is monitored by NMR. 225 ml of water is then added and the mixture then divided into two portions.

(a) One portion is acidified to pH 3 with dilute aqueous hydrochloric acid and extracted with methylene chloride. The methylene chloride extracts are combined, dried with magnesium sulfate and evaporated to afford 4-methoxycarbonyl-5-(2-ethylthiopropyl)-1,3-cyclohexanedione as the residue.

(b) The other portion of the watered reaction product mixture is treated with an equivalent amount of 1N aqueous sodium hydroxide solution at room to hydrolyze off the methoxy group. After hydrolysis is complete, the mixture is heated to 50-60 °C and acidified with aqueous 18 wt. % hydrochloric acid to decarboxylate the 4-position thereby affording the title compound.

Similarly, by applying this procedure to the compounds listed in Example 2, the corresponding 5-substituted-4-methoxycarbonyl-1,3-cyclohexanediones can be prepared.

## Claims

**1.** A process for preparing a 4,5-disubstituted-1,3-cyclohexanedione represented by the general formula:

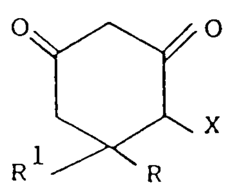

wherein X is cyano, nitro or $COOR^b$ where $R^b$ is lower alkyl; R is lower alkyl, cycloalkyl having 3 to 7

carbon atoms, lower alkoxy, haloalkyl having 1 or 2 carbon atoms and 1 to 3 of the same or different halo atoms, aryl having 6 to 10 carbon atoms; substituted aryl having 6 to 10 carbon atoms in the aryl moiety and having 1 to 3 substituents independently selected from lower alkyl, lower alkenyl, lower alkoxy, lower alkylthio, halo, haloalkyl having 1 or 2 carbon atoms and 1 to 3 of the same or different halo atoms, and nitro; arylalkyl having 1 to 4 carbon atoms in the alkyl moiety and 6 to 10 carbon atoms in the aryl moiety; substituted arylakyl wherein the alkyl moiety has 1 to 4 carbon atoms and the substituted aryl moiety is as defined hereinabove; monocyclic heterocyclic or heterocyclic-alkyl having 1 to 4 carbon atoms in the alkyl moiety and wherein the heterocyclic moiety has 5-or 6-ring atoms, 1 or 2 of which are independently oxygen, sulfur or nitrogen atoms, and the remainder are carbon atoms; or R is the radical $R^5S(0)_nR^4$ - where $R^4$ is lower alkylene, $R^5$ is lower alkyl, lower alkenyl, cycloalkyl as defined hereinabove, aryl, substituted aryl as defined hereinabove, or arylalkyl or substituted arylalkyl as defined hereinabove and n is 0, 1 or 2; and $R^1$ is hydrogen or a substituent as defined for R, or R and $R^1$ together with the carbon atom to which they are joined form a cycloalkylidene group having 5 to 7 carbon atoms, which comprises reacting in a known manner an $\alpha,\beta$-unsaturated ketone of the general formula:

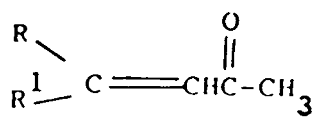

wherein R and $R^1$ have the meanings hereinbefore defined, with a substituted acetate ester of the general formula:

$XCH_2\ COOR^a$

wherein $R^a$ is lower alkyl and X has the meaning hereinbefore defined, in the presence of an alkali metal lower alkoxide to obtain the desired compound, if necessary by means of a decarboxylation step, characterised in that the $\alpha,\beta$ -unsaturated ketone starting compound is produced by a process which comprises the separate steps of:

(a) contacting an aldehyde or ketone of the general formula:

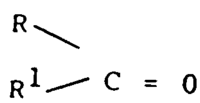

wherein R and $R^1$ have the meanings hereinbefore defined, with an acetoacetate salt in the presence of a catalytic amount of a tertiary amine to form a compound of the general formula:

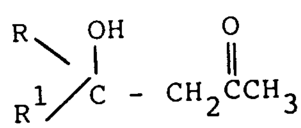

wherein R and $R^1$ have the meanings hereinbefore defined, and thereafter

(b) contacting said compound with a dehydration catalyst at a temperature in the range from 60 to 200°C to produce the required $\alpha,\beta$-unsaturated starting compound.

**2.** A process according to Claim 1, wherein $R^1$ is hydrogen and R is the radical $R^5S(0)_nR^4$- in which $R^4$, $R^5$ and n are as defined in Claim 1.

**3.** A process according to Claim 2, wherein $R^5$ is lower alkyl.

**4.** A process according to Claim 3, wherein R has the formula:

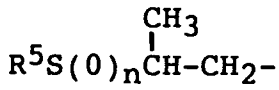

wherein $R^5$ is lower alkyl and n is 0, 1 or 2.

**5.** A process according to Claim 4, wherein R is 2-ethylthiopropyl and the $\alpha,\beta$ -unsaturated starting compound obtained is 6-ethylthiohept-3-en-2-one.

## Revendications

**1.** Procédé de préparation d'une 1,3-cyclohexanedione disubstituée en positions 4,5, représentée par la formule générale :

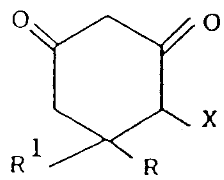

dans laquelle X représente un groupe cyano, nitro ou $COOR^b$ dans lequel $R^b$ représente un groupe alkyle inférieur ; R représente un groupe alkyle inférieur, cycloalkyle ayant 3 à 7 atomes de carbone, alkoxy inférieur, halogénalkyle ayant 1 ou 2 atomes de carbone et 1 à 3 atomes d'halogènes identiques ou différents, aryle ayant 6 à 10 atomes de carbone ; aryle substitué ayant 6 à 10 atomes de carbone dans le groupement aryle et portant 1 à 3 substituants choisis indépendamment entre des groupes alkyle inférieur, alcényle inférieur, alkoxy inférieur, alkylthio inférieur, halogéno, halogénalkyle ayant 1 ou 2 atomes de carbone et 1 à 3 atomes d'halogènes identiques ou différents, et nitro ; alkylaryle ayant 1 à 4 atomes de carbone dans le groupement alkyle et 6 à 10 atomes de carbone dans le groupement aryle ; alkylaryle substitué dans lequel le groupement alkyle possède 1 à 4 atomes de carbone et le groupement aryle substitué répond à la définition précitée ; hétérocyclique monocyclique ou alkyle hétérocyclique ayant 1 à 4 atomes de carbone dans le groupement alkyle et dans lequel le groupement hétérocyclique possède 5 ou 6 atomes dans le noyau, 1 ou 2 de ces atomes étant choisis indépendamment entre des atomes d'oxygène, de soufre et d'azote et le reste consistant en atomes de carbone ; ou bien R représente le radical $R^5S(O)_nR^4$ - dans lequel $R^4$ représente un groupe alkylène inférieur, $R^5$ représente un groupe alkyle inférieur, alcényle inférieur, cycloalkyle répondant à la définition précitée, aryle, aryle substitué répondant à la définition précitée ou arylalkyle ou arylalkyle substitué répondant à la définition précitée et n a la valeur 0, 1 ou 2 ; et $R^1$ représente l'hydrogène ou un substituant répondant à la définition mentionnée pour R, ou bien R et $R^1$, conjointement avec l'atome de carbone auquel ils sont liés, forment un groupe cycloalkylidène ayant 5 à 7 atomes de carbone, qui consiste à faire réagir d'une manière connue une cétone $\alpha,\beta$-insaturée de formule générale :

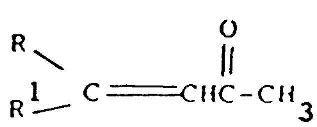

dans laquelle R et $R^1$ répondent aux définitions précitées, avec un ester acétique substitué de formule générale :

$XCH_2\ COOR^a$

dans laquelle $R^a$ représente un groupe alkyle inférieur et X répond à la définition précitée, en présence d'un alcoolate inférieur de métal alcalin pour obtenir le composé désiré, si nécessaire au moyen d'une étape de décarboxylation, caractérisé en ce que la cétone $\alpha,\beta$-insaturée servant de composé de départ est produite par un procédé qui comprend les étapes distinctes consistant :

(a) à mettre en contact un aldéhyde ou une cétone de formule générale :

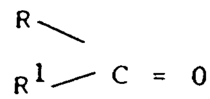

dans laquelle R et $R^1$ répondent aux définitions précitées, avec un acétoacétate en présence d'une quantité catalytique d'une amine tertiaire pour former un composé de formule générale :

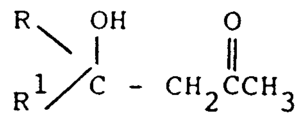

dans laquelle R et $R^1$ répondent aux définitions précitées, puis
(b) à mettre en contact ledit composé avec un catalyseur de déshydratation à une température comprise dans l'intervalle de 60 à 200° C pour produire le composé $\alpha,\beta$-insaturé de départ requis.

2. Procédé suivant la revendication 1, dans lequel $R^1$ représente l'hydrogène et R représente le radical $R^5S(O)_nR^4$- dans lequel $R^4$, $R^5$ et n répondent aux définitions suivant la revendication 1.

3. Procédé suivant la revendication 2, dans lequel $R^5$ représente un groupe alkyle inférieur.

4. Procédé suivant la revendication 3, dans lequel R répond à la formule :

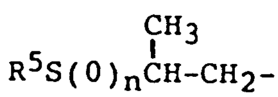

dans laquelle $R^5$ représente un groupe alkyle inférieur et n a la valeur 0, 1 ou 2.

5. Procédé suivant la revendication 4, dans lequel R représente un groupe 2-éthylthiopropyle et le composé de départ $\alpha,\beta$-insaturé obtenu est la 6-éthylthiohept-3-ène-2-one.

**Patentansprüche**

1. Verfahren zur Herstellung eines 4,5-disubstituierten 1,3-Cyclohexandions der allgemeinen Formel

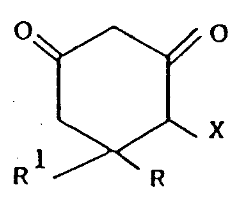

wobei

X die Bedeutung Cyano, Nitro oder $COOR^b$ hat, wobei $R^b$ niederes Alkyl bedeutet;

R bedeutet;

niederes Alkyl, Cycloalkyl mit 3 bis 7 Kohlenstoffatomen, niederes Alkoxy, Haloalkyl mit 1 oder 2 Kohlenstoffatomen und 1 bis 3 der gleichen oder unterschiedlichen Halogenatome, Aryl mit 6 bis 10 Kohlenstoffatomen; substituiertes Aryl mit 6 bis 10 Kohlenstoffatomen im Arylanteil und mit 1 bis 3 Substituenten, die unabhängig ausgewählt sind aus niederem Alkyl, niederem Alkenyl, niederem Alkoxy, niederem Alkylthio, Halo, Haloalkyl mit 1 oder 2 Kohlenstoffatomen und 1 bis 3 der gleichen oder unterschiedlichen Halogenatomen, und Nitro;

Arylalkyl mit 1 bis 4 Kohlenstoffatomen im Alkylanteil und 6 bis 10 Kohlenstoffatomen im Arylanteil; substituiertes Arylalkyl, wobei der Alkylanteil 1 bis 4 Kohlenstoffatome hat und der substituierte Arylanteil wie vorstehend definiert ist; monocyclisches heterocyclisches oder heterocyclisches Alkyl mit 1 bis 4 Kohlenstoffatomen im Alkylanteil, wobei der heterocyclische Anteil 5- oder 6-Ringatome hat, von denen 1 oder 2 unabhängig Sauerstoff-, Schwefel- oder Stickstoffatome sind und der Rest Kohlenstoffatome ist; oder R den Rest $R^5S(O)_nR^4$-bedeutet, wobei $R^4$ niedriges Alkylen bedeutet, $R^5$ niedriges Alkyl, niedriges Alkenyl, wie vorstehend definiertes Cycloalkyl, Aryl, wie vorstehend definiertes substituiertes Aryl oder Arylalkyl oder wie vorstehend definiertes substituiertes Arylalkyl bedeutet und n die Bedeutung 0,1 oder 2 hat; und

$R^1$ Wasserstoff oder einen wie für R definierten Substituenten bedeutet; oder R und $R^1$ zusammen mit den Kohlenstoffatom, mit dem sie verbunden sind, eine Cycloalkylidiengruppe mit 5 bis 7 Kohlenstoffatomen bilden;

wobei man in bekannter Art ein $\alpha,\beta$-ungesättigtes Keton der allgemeinen Formel

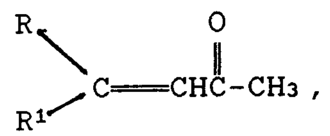

wobei R und $R^1$ die vorstehend definierten Bedeutungen haben, mit einem substituierten Acetatester der allgemeinen Formel

$XCH_2COOR^a$

zum Erhalten der gewünschten Verbindung in Gegenwart eines niederen Alkalimetallalkoxids, falls erforderlich mit Hilfe eines Decarboxylierungsschrittes, umsetzt, wobei $R^a$ niederes Alkyl bedeutet und X die vorstehend definierten Bedeutungen hat,

**dadurch gekennzeichnet,**

daß die $\alpha,\beta$-ungesättigte Keton-Ausgangsverbindung hergestellt wird mit einem Verfahren, welches die getrennten Schritte enthält:

(a) Kontaktieren eines Aldehyds oder Ketons der allgemeinen Formel

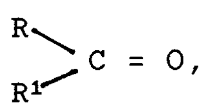

wobei R und $R^1$ die vorstehend definierten Bedeutungen haben, mit einem Acetoacetatsalz in Gegenwart einer katalytischen Menge eines tertiären Amins zur Bildung einer Verbindung der allgemeinen Formel

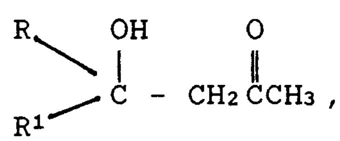

wobei R und $R^1$ die vorstehend definierten Bedeutungen haben, und danach

(b) Kontaktieren der Verbindung mit einem Dehydrierungskatalysator bei einer Temperatur im Bereich von 60 bis 200 °C zum Erzeugen der gewünschten $\alpha,\beta$-ungesättigten Ausgangsverbindung.

**2.** Verfahren nach Anspruch 1, wobei $R^1$ die Bedeutung Wasserstoff hat und R den Rest $R^5S(O)nR^4$-bedeutet, wobei $R^4$, $R^5$ und n die in Anspruch 1 definierte Bedeutungen haben.

**3.** Verfahren nach Anspruch 2, wobei $R^5$ niederes Alkyl bedeutet.

**4.** Verfahren nach Anspruch 3, wobei R die Formel

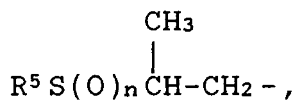

hat, wobei $R^5$ niederes Alkyl bedeutet und n die Bedeutung 0,1 oder 2 hat.

**5.** Verfahren nach Anspruch 4, wobei R die Bedeutung 2-Ethylthiopropyl hat und die erhaltene $\alpha,\beta$-ungesättigte Ausgangsverbindung 6-Ethylthiohept-3-en-2-on ist.